# EUROPÄISCHE PATENTSCHRIFT

(11) **EP 4 307 945 B1**
(45) Veröffentlichungstag und Bekanntmachung des Hinweises auf die Patenterteilung: **12.11.2025**
(21) Anmeldenummer: 22715065.3
(22) Anmeldetag: 16.03.2022
(51) Int. Cl.: A24F 40/44, A24F 40/48, A61M 11/04, A24F 40/42, A24F 40/485, A24F 40/51, A61M 15/06

(54) **VERDAMPFEREINRICHTUNG**
EVAPORATOR DEVICE
DISPOSITIF D'ÉVAPORATEUR

(30) Priorität: 16.03.2021 DE 102021202544
(43) Veröffentlichungstag der Anmeldung: 24.01.2024
(73) Patentinhaber: Alveon GmbH, 8807 Freienbach (CH)
(72) Erfinder: GEILEN, André, 8807 Freienbach (CH); GEISS, Simon, 8807 Freienbach (CH); RIESS, Ralf Martin, 8807 Freienbach (CH)
(74) Vertreter: BRP Renaud & Partner mbB Rechtsanwälte Patentanwälte Steuerberater
(86) Internationale Anmeldenummer: PCT/EP2022/056786
(87) Internationale Veröffentlichungsnummer: WO 2022/194914

(56) Entgegenhaltungen:
- EP-A1- 2 468 118
- WO-A1-2010/118644
- US-A1- 2015 237 916
- US-A1- 2017 196 273
- US-A1- 2017 280 779

## Beschreibung

Die vorliegende Erfindung betrifft eine Verdampfereinrichtung zum Verdampfen einer Substanz sowie einen Inhalator mit einer solchen Verdampfereinrichtung.

Eine Verdampfereinrichtung dient in der Regel dem Verdampfen einer Flüssigkeit und kommt in üblicherweise in Inhalatoren zum Einsatz. Gewöhnlich weist eine solche Verdampfereinrichtung einen Docht, der mit der zu verdampfenden Flüssigkeit getränkt wird, sowie einen Heizdraht auf, der wärmeübertragend mit dem Docht verbunden ist und bei elektrischer Versorgung Wärme erzeugt, um die im Docht gespeicherte Flüssigkeit zu verdampfen.

Aus der DE 10 2018 119 566 A1 ist eine Verdampfereinrichtung mit einem Verdampfer aus einem elektrisch leitfähigen porösen Element bekannt, welches zugleich zur zum Speichern der Flüssigkeit und zum Heizen zwecks Verdampfen der Flüssigkeit zum Einsatz kommt, um die Bildung von sogenannten Dry Hits, das heißt lokale, übermäßige Erwärmungen, welche zur Zerstörung des Dochts führen können, zu vermeiden.

Aus der DE 10 2016 120 803 A1 ist eine Verdampfereinrichtung mit einer aus einem dotierten und elektrisch leitfähigen Verdampferkeramik bestehendem Verdampfer bekannt. Die Verdampferkeramik ist mit geregelten und eine vorgegebene Orientierung aufweisenden Mikrokanälen versehen, durch welche Flüssigkeit zwecks Verdampfens strömt. Die Verdampferkeramik erzeugt hierbei bei elektrischer Versorgung Wärme, um die darin aufgenommene Flüssigkeit zu verdampfen. Die Verdampfereinrichtung weist ferner eine Durchflusssteuerungseinrichtung auf, welche den Durchfluss der Flüssigkeit durch die Mikrokanäle steuert, um eine Dosierung der zu verdampfenden Flüssigkeit zu erreichen.

Aus der DE 10 2017 123 868 A1 ist eine Verdampfereinrichtung bekannt, welche wie die aus der DE 10 2016 120 803 A1 bekannte Verdampfereinrichtung eine Verdampferkeramik mit parallelen Mikrokanälen zum Speichern und Verdampfen der Flüssigkeit aufweist. Um eine Blasenbildung im Einlassbereich der Mikrokanäle zu vermeiden, ist im Einlassbereich zusätzlich eine Dochtstruktur vorgesehen und flächig kontaktierend mit der Einlassseite verbunden.

Weitere Verdampfungseinrichtungen und Inhalatoren sind aus der WO 2010/118644 A1, der US 2017/196273 A1, der US 2017/280779 A1, der EP 2 468 118 A1 sowie der US 2015/237916 A1 bekannt.

Nachteilig bei aus dem Stand der Technik bekannten Verdampfereinrichtungen ist die komplizierte Herstellung sowie die mangelnde und/oder aufwändige Kontrolle der Verdampfung der zu verdampfenden Flüssigkeit.

Die vorliegende Erfindung beschäftigt sich daher mit der Aufgabe, für eine Verdampfereinrichtung sowie für einen Inhalator mit einer solchen Verdampfereinrichtung verbesserte oder zumindest andere Ausführungsformen anzugeben, welche sich insbesondere durch eine vereinfachte Herstellung und/oder verbesserte Kontrolle der Verdampfung auszeichnen.

Diese Aufgabe wird erfindungsgemäß durch den Gegenstand des unabhängigen Anspruchs gelöst. Vorteilhafte Ausführungsformen sind Gegenstand der abhängigen Ansprüche.

Die vorliegende Offenbarung beruht auf dem allgemeinen Gedanken, eine Verdampfereinrichtung mit einer eine Aufnahmestruktur aufweisenden elektrisch leitfähigen Keramik bereitzustellen, wobei die Aufnahmestruktur der Aufnahme einer zu verdampfenden Substanz dient, und wobei die elektrisch leitfähige Keramik zugleich der Erzeugung von Wärme zum Verdampfen der in der Aufnahmestruktur aufgenommenen Substanz dient, und einen elektrischen Leiter zur elektrischen Versorgung der elektrisch leitfähigen Keramik vorzusehen, der in einem Pfad des elektrischen Stroms zur elektrischen Versorgung der elektrisch leitfähigen Keramik angeordnet und wärmeübertragend mit der elektrisch leitfähigen Keramik verbunden ist, wobei der Leiter beim Überschreiten einer vorgegebenen Temperatur einen sprunghaft ansteigenden elektrischen Widerstand aufweist. Die Verwendung der Aufnahmestruktur der elektrisch leitfähigen Keramik bei zu gleichem Einsatz der elektrisch leitfähigen Keramik als elektrischer Heizer zum Verdampfen der Substanz führen zu einer vereinfachten Herstellung der Verdampfereinrichtung und erlauben das kontrollierte Verdampfen von Substanz, insbesondere eines kontrollierbaren Volumens der Substanz. Der sprunghafte Anstieg des elektrischen Widerstands des mit der elektrisch leitfähigen Keramik wärmeübertragend verbundenen elektrischen Leiters beim Überschreiten der vorgegebenen Temperatur führt ferner dazu, dass eine elektrische Versorgung der elektrisch leitfähigen Keramik beim Erreichen der vorgegebenen Temperatur unterbrochen oder zumindest derart reduziert wird, dass die vorgegebene Temperatur eine maximale Temperatur der elektrisch leitfähigen Keramik vorgibt. Dies führt auf besonders einfache und effektive Weise dazu, dass die elektrisch leitfähige Keramik im Betrieb die vorgegebene Temperatur nicht überschreitet, sodass eine genau definierte Kontrolle der Verdampfungsparameter gegeben ist. Ferner ist es auf diese Weise möglich, die Verdampferkeramik mit einer Leistung zu betreiben, welche ohne den Sperrleiter zu einer Überhitzung führen würde. Dadurch ist es ohne aufwendige Regelung möglich, die Verdampferkeramik schnell und mit hoher Leistung auf Temperaturen im Betriebsbereich zu bringen und im Betriebsbereich zu halten.

Dem Erfindungsgedanken entsprechend weist die Verdampfereinrichtung den Verdampfer auf. Der Verdampfer umfasst die elektrisch leitfähige Keramik, die nachfolgend auch als Verdampferkeramik bezeichnet wird. Die Verdampferkeramik dient zugleich dem Aufnehmen und Speichern der zu verdampfenden Substanz und dem Erzeugen von Wärme zum Verdampfen der Substanz. Das Speichern der Substanz erfolgt mittels der Aufnahmestruktur der Verdampferkeramik. Die Verdampferkeramik weist also die Aufnahmestruktur auf, in welcher im Betrieb die zu verdampfende Substanz aufgenommen ist. Zur elektrischen Versorgung des Verdampfers, insbesondere der Verdampferkeramik, weist die Verdampfereinrichtung ferner zwei elektrische Anschlüsse auf. Zwischen den beiden elektrischen Anschlüssen sowie durch die Verdampferkeramik verläuft der Pfad des elektrischen Stroms, nachfolgend auch Strompfad genannt. Die Verdampferkeramik ist derart ausgestaltet, dass sie mittels ihrer elektrisch leitfähigen Eigenschaft im Betrieb bei elektrischer Versorgung in der Verdampferkeramik homogen Wärme erzeugt, um die in der Aufnahmestruktur aufgenommene Substanz zu verdampfen. Der Verdampfer, insbesondere die Verdampferkeramik, ist für den Betrieb in einem thermischen Betriebsbereich ausgelegt, der von einer unteren Betriebsanfangstemperatur und einer oberen Betriebsendtemperatur begrenzt ist. Mit anderen Worten, zum Verdampfen der Substanz erzeugt die Verdampferkeramik bei elektrischer Versorgung Wärme im Betriebsbereich und somit zwischen der Betriebsanfangstemperatur und der Betriebsendtemperatur. Die homogene Wärmeerzeugung der Verdampferkeramik führt zu einer homogenen Temperatur bzw. einer homogenen Wärmeverteilung im Volumen der Verdampferkeramik und folglich in der Aufnahmestruktur. Dies führt zu einer gleichmäßigen Verdampfung der Substanz in der gesamten Aufnahmestruktur. Die Verdampfereinrichtung weist ferner den vorstehend erwähnten elektrischen Leiter auf, der nachfolgend auch als Sperrleiter bezeichnet wird. Der Sperrleiter ist erfindungsgemäß im Strompfad angeordnet, sodass der elektrische Strom im Betrieb durch den Sperrleiter fließt. Zudem ist der Sperrleiter derart ausgestaltet, dass er bei der Betriebsendtemperatur einen sprunghaft ansteigenden elektrischen Widerstand aufweist. Durch die wärmeübertragende Verbindung des Sperrleiters mit der Verdampferkeramik erfolgt in der Folge beim Erreichen der Betriebsendtemperatur ein sprunghafter Anstieg des elektrischen Widerstands im Strompfad und somit eine Unterbrechung oder zumindest erhebliche Reduzierung der elektrischen Versorgung der Verdampferkeramik. Dementsprechend wird die Betriebsendtemperatur mittels des Sperrleiters zumindest im Wesentlichen beeinflusst, vorzugsweise bestimmt.

Die wärmeübertragende Verbindung des Sperrleiters mit der Verdampferkeramik ist vorteilhaft derart, dass die Temperatur des Sperrleiters zumindest im Wesentlichen der Temperatur der Verdampferkeramik entspricht.

Die Verdampferkeramik erzeugt, wie erwähnt, bei elektrischer Versorgung mittels ihrer elektrischen Leitfähigkeit homogen Wärme. Insbesondere ist die Verdampferkeramik ein Heizwiderstand.

Die Verdampferkeramik kann eine elektrisch leitfähige Keramik beliebiger Art sein, sofern sie die Aufnahmestruktur aufweist und bei elektrischer Versorgung, insbesondere beim Anliegen einer elektrischen Spannung, in einem vorgegebenen Bereich, homogen Wärme im Betriebsbereich erzeugt.

Vorstellbar ist es, dass die Verdampferkeramik per se elektrisch leitfähig ist. Hierzu zählen beispielsweise Keramiken aus Metalloxiden, wie Titan-Oxide, oder Metallcarbide sowie Silizium-Carbide. Ebenso können Komposit-Keramiken zum Einsatz kommen, welche elektrisch leitfähige und elektrisch nicht leitfähige Netzwerke unterschiedlicher Werkstoffe aufweisen, wobei die leitfähigen Netzwerke zweckmäßig homogen in der Keramik verteilt sind. Beispiele für solche Komposit-Keramiken sind solche mit Metalloxiden unterschiedlicher Oxidationsstufe. Auch können Mischoxid-Keramiken zum Einsatz kommen, welche durch Vermengen unterschiedlicher Ausgangwerkstoffe hergestellt werden, wobei beim Herstellen der Keramik, typischerweise beim Sintern, durch chemische Reaktionen ein neuer Werkstoff entsteht. Beispiele für die Ausgangswerkstoffe sind unterschiedliche Metalloxide. Ferner können dotierte Keramiken zum Einsatz kommen, welche durch Dotierung elektrisch leitfähig werden. Selbstverständlich können auch beliebige Kombinationen der genannten Keramiken zum Einsatz kommen, sofern die Verdampferkeramik eine elektrische leitfähige Keramik mit der Aufnahmestruktur ist, welche im Betrieb homogen Wärme erzeugt.

Die Verdampfereinrichtung lässt sich sowohl kontinuierlich als auch diskontinuierlich betrieben. Entsprechend ist die Verdampfereinrichtung ausgestaltet.

Beim kontinuierlichen Betrieb wird die Verdampferkeramik für zumindest eine begrenzte Dauer kontinuierlich mit Substanz versorgt und verdampft diese Substanz zumindest teilweise. Die Versorgung mit der Substanz kann durch eine dauerhaft fluidische Verbindung der Verdampfereinrichtung, insbesondere der Verdampferkeramik, mit einem Substanzbehälter, in welchem die Substanz bevorratet ist, realisiert sein, so dass Substanz kontinuierlich in die Verdampferkeramik nachströmt. Die Verdampfung erfolgt dabei solange die Verdampfereinrichtung elektrisch versorgt wird und im Betriebsbereich betrieben ist. Die verdampfte Menge der Substanz kann dabei bei Bedarf über die Betriebsdauer der Verdampfereinrichtung im Betriebsbereich kontrolliert werden.

Beim diskontinuierlichen Betrieb wird der Verdampferkeramik eine vorgegebene Dosis der Substanz zugeführt, welche dann verdampft wird. Insbesondere ist beim diskontinuierlichen Betrieb die Verdampfereinrichtung, insbesondere die Verdampferkeramik, nicht kontinuierlich mit der Substanz versorgt. Somit lässt sich die verdampfte Menge insbesondere über das Volumen der Verdampferkeramik und/oder die in der Verdampferkeramik aufgenommene Menge der Substanz kontrollieren.

Beim diskontinuierlichen Betrieb kann der Verdampfereinrichtung, insbesondere der Verdampferkeramik, Substanz zugeführt werden, nachdem die zuvor in der Verdampferkeramik aufgenommene Substanz zumindest teilweise verdampft und/oder die Verdampfereinrichtung nicht im Betriebsbereich betrieben, insbesondere außer Betrieb, ist. Die Verdampfereinrichtung, insbesondere die Verdampferkeramik, ist also nachfüllbar.

Beim diskontinuierlichen Betrieb ist es ebenso vorstellbar, die Verdampfereinrichtung bereits mit einer Dosis der Substanz zu bevorraten, die im Betrieb verdampft wird. Die Verdampfereinrichtung kann hierbei für den einmaligen Gebrauch ausgestaltet, also austauschbar sein. Insbesondere kann die Verdampfereinrichtung in der Art einer Tablette ausgestaltet sein.

Selbstverständlich sind auch Mischbetriebe aus diskontinuierlichem und kontinuierlichem Betrieb möglich.

Die Aufnahmestruktur ist vorteilhaft integral in der Verdampferkeramik ausgebildet und/oder ausgeformt.

Die Aufnahmestruktur ist zweckmäßig homogen in der Verdampferkeramik verteilt.

Vorstellbar ist es, die Aufnahmestruktur durch nachträgliche Bearbeitung in die Verdampferkeramik einzubringen. Insbesondere ist es denkbar, in die Verdampferkeramik Kanäle, beispielsweise Mikrokanäle auszuformen, welche Bestandteil der Aufnahmestruktur sind oder die Aufnahmestruktur bilden.

Bevorzugt weist die Aufnahmestruktur Poren in der Verdampferkeramik auf. Besonders bevorzugt besteht die Aufnahmestruktur aus Poren, ist also eine Porenstruktur.

Die Poren der Verdampferkeramik sind vorteilhaft bei der Herstellung der Verdampferkeramik, welche beispielsweise mittels Sintern erfolgen kann, ausgebildet. Mit anderen Worten, die Poren zum Aufnehmen der Substanz sind vorteilhaft nicht gesondert, insbesondere nicht nachträglich, in die Verdampferkeramik eingebracht. Somit erfolgt die Verwendung einer intrinsischen, durch die Herstellung gegebenen, Eigenschaft der Verdampferkeramik zum Speichern der zu verdampfenden Substanz. Dies führt zu einer einfachen und kostengünstigen Herstellung der Verdampferkeramik und somit der Verdampfereinrichtung.

Zudem lässt sich durch die Herstellung der Verdampferkeramik ein durch die Poren definiertes Gesamtvolumen der Verdampferkeramik und somit Volumen der aufnehmbaren Substanz variieren und vorgeben. Dies führt zu einer weiteren, einfach ausgestalteten Kontrolle der Verdampfungsparameter.

Die Verdampferkeramik kann prinzipiell Poren beliebiger Art aufweisen.

Vorteilhaft ist es, wenn die Verdampferkeramik Poren mit einer mittleren Größe zwischen 0,05 µm und 50 µm aufweist. Diese mittleren Porengrößen führen bei einer Flüssigkeit als Substanz zu einem derartigen Verhältnis zwischen der Oberfläche und dem Volumen der jeweiligen Pore, dass diese kapillaren Kräfte aufweisen, welche die gravitationsbedingt und/oder druckbedingt auf ein in dem Volumen aufgenommenes tropfenförmiges Teilchen der Flüssigkeit wirkenden Kräfte ausgleichen, vorzugsweise überwiegen. Daraus resultiert, dass die tropfenförmigen Teilchen, nachfolgend auch als Tröpfchen bezeichnet, in den Poren verbleiben. Folglich ist ein Abfließen der Tröpfchen und folglich der Flüssigkeit aus der Verdampferkeramik verhindert oder zumindest erheblich reduziert. Somit lassen sich in der Verdampferkeramik auch niederviskose Flüssigkeiten aufnehmen und speichern. Somit ist es mit der Verdampferkeramik also möglich, eine größere Variabilität an Flüssigkeiten unterschiedlicher Viskosität aufzunehmen und zu speichern, ohne dass die Flüssigkeiten aus der Verdampferkeramik abfließen. In der Folge können die Flüssigkeiten kostengünstiger und in einem breiteren Spektrum bereitgestellt werden. Insbesondere können somit in den Flüssigkeiten aufgenommene Wirkstoffe vereinfacht und/oder mit einer genaueren Dosis bereitgestellt werden. Somit lassen sich die Verdampferkeramik und die zugehörige Verdampfereinrichtung vereinfacht für eine kontrollierbare Inhalation besagter Wirkstoffe und somit eine kontrollierbare und/oder vorgegebene Dosierung der Wirkstoffe einsetzen. Die vorstehend beschriebenen kapillaren Kräfte führen ferner dazu, dass sich die Verdampferkeramik bei einer hydraulischen Verbindung mit der zur verdampfenden Flüssigkeit ohne weitere Einwirkung, wie beispielsweise ein aktives Pumpen der Flüssigkeit in die Verdampferkeramik, mit der Flüssigkeit vollsaugt. Insgesamt können somit gesonderte Abdichtungen der Verdampferkeramik entfallen oder zumindest reduziert werden und/oder Einrichtungen zum aktiven Einbringen der Flüssigkeit in die Keramik entfallen. Somit lassen sich sowohl die Verdampferkeramik als auch eine zugehörige Verdampfereinrichtung einfach und kostengünstig umsetzen. Somit erfolgt also neben einer Erhöhung der Einsatzmöglichkeiten der Verdampferkeramik sowie der zugehörigen Verdampfereinrichtung eine vereinfachte Umsetzung derselben.

Ein weiterer Vorteil der besagten mittleren Porengrößen ist darin zu sehen, dass diese zu einer Vergrößerung der mit der Verdampferkeramik in Kontakt stehenden Fläche der Tröpfchen führen. Mit anderen Worten, eine vergrößerte Fläche der Verdampferkeramik überträgt zum Verdampfen der Flüssigkeit Wärme auf die Tröpfchen. Dies führt zu einer gleichmäßigeren Verdampfung der Flüssigkeit und somit einer verbesserten Kontrolle über die Verdampfung. Zudem kommt es auf diese Weise zu einer schnelleren Verdampfung der Flüssigkeit.

Unter mittlere Porengröße ist vorliegend insbesondere das Verhältnis zwischen dem vierfachen Volumen und der Fläche der Poren, also 4V/A, wie dies insbesondere in der Norm ISO 15901 angegeben ist, zu verstehen.

Wie vorstehend beschrieben, kann die Verdampferkeramik insbesondere zur Aufnahme von niederviskosen Flüssigkeiten zum Einsatz kommen. Unter niederviskose Flüssigkeiten sind dabei insbesondere Flüssigkeiten zu verstehen, welche eine Viskosität von 45 mPas und kleiner aufweisen.

Bei der Flüssigkeit kann es sich um eine beliebige Flüssigkeit handeln. Insbesondere ist es möglich medizinische Wirkstoffe enthaltende Flüssigkeiten einzusetzen.

Bevorzugt ist es, wenn die Porengrößen der Poren der Porenstruktur zumindest größtenteils innerhalb der mittleren Porengröße liegen. Das heißt insbesondere, dass maximal 10 % der Poren Porengrößen aufweisen, welche größer sind als das 4-fache der mittleren Porengröße. Dies führt dazu, dass Poren mit Porengrößen oberhalb der mittleren Porengröße reduziert, vorzugsweise nicht, vorhanden sind. In der Folge sind die Auswirkungen von Poren mit Porengrößen oberhalb der mittleren Porengröße auf das Gesamtverhalten der Verdampferkeramik und folglich die Auswirkungen von Tröpfchen mit größeren Volumen in diesen Poren auf das Gesamtverhalten der in der Verdampferkeramik aufgenommenen Flüssigkeit vernachlässigbar oder zumindest reduziert. Somit lässt sich insbesondere verhindern, dass die Flüssigkeit aus der Verdampferkeramik abfließt. Dies führt ferner dazu, dass die in den Poren aufgenommenen Tröpfchen entsprechen der Größenverteilung der Poren im Wesentlichen gleiche Volumen aufweisen. Dies führt zu einer homogenen Verteilung der in der Verdampferkeramik aufgenommenen Flüssigkeit über das Volumen der Keramik. Darüber hinaus lässt sich die Flüssigkeit auf diese Weise homogener und/oder kontrollierter verdampfen.

Als vorteilhaft gelten Ausführungsformen, bei denen die mittlere Porengröße zwischen 0,1 µm und 25 µm, bevorzugt zwischen 0,15 µm und 10 µm, besonders bevorzugt zwischen 0,2 µm und 5 µm beträgt. Auf diese Weise kommt es zu einer vorteilhaften Wechselwirkung zwischen der in den Poren aufgenommenen Tröpfchen, den kapillaren Kräften sowie der Verteilung der Flüssigkeit im Volumen der Verdampferkeramik, welche zu einer verbesserten Aufnahme der Flüssigkeit in der Verdampferkeramik sowie einer verbesserten Verdampfung der in der Verdampferkeramik aufgenommenen Flüssigkeit führen.

Der Strompfad der Verdampfereinrichtung ist zweckmäßig derart, dass der elektrische Strom bei elektrischer Versorgung zwingend entlang des Strompfads strömt. Das heißt insbesondere, dass eine elektrische Umgehung des Sperrleiters nicht möglich und/oder nicht vorgesehen ist.

Bei der Substanz handelt es sich um eine solche, die durch Erhitzen verdampft. Die Substanz ist also verdampfbar.

Die Substanz ist vorteilhaft flüssig. Insbesondere ist die Substanz eine Flüssigkeit. Denkbar sind sowohl zähflüssige als auch dünnflüssige Flüssigkeiten, also Flüssigkeiten mit unterschiedlichen Viskositäten.

Die Verdampfereinrichtung kann prinzipiell einen einzigen Sperrleiter aufweisen.

Vorstellbar ist es auch, die Verdampfereinrichtung mit zwei oder mehr solchen Sperrleitern zu versehen. Bevorzugt ist es hierbei, wenn die Sperrleiter identisch sind.

Unter einem sprunghaften Anstieg des elektrischen Widerstands beim Überschreiten der Betriebsendtemperatur ist vorliegend ein derartiger Anstieg zu verstehen, der einen linearen Anstieg übersteigt.

Bevorzugt ist es, wenn zumindest einer der wenigstens einen Sperrleiter beim Überschreiten der Betriebsendtemperatur einen potenziellen Anstieg des elektrischen Widerstands zeigt. Besonders bevorzugt sind Ausführungsformen, bei denen zumindest einer der wenigstens einen Sperrleiter derart ausgestaltet ist, dass sein elektrischer Widerstand beim Überschreiten der Betriebsendtemperatur anfängt, exponentiell anzusteigen. Als vorteilhaft erweisen sich Ausführungsformen, bei denen der elektrische Widerstand zumindest eines der wenigstens einen Sperrleiter, vorteilhaft des jeweiligen Sperrleiters, in den auf die Betriebsendtemperatur folgenden 50°C um zumindest eine Zehnerpotenz steigt. Somit lassen sich die Verdampfungsparameter besonders einfach und effektiv kontrollieren.

Als vorteilhaft gelten Ausführungsformen, bei denen zumindest einer der wenigstens einen Sperrleiter, vorteilhaft der jeweilige Sperrleiter, als ein Kaltleiter ausgestaltet ist, wobei die Betriebsendtemperatur zwischen einer Anfangstemperatur und einer Endtemperatur des zumindest einen Kaltleiters liegt. Kaltleiter weisen eine charakteristische Strom-Kennlinie auf, wobei der elektrische Widerstand ab der Anfangstemperatur sprunghaft um mehrere Zehnerpotenzen steigt. Auf diese Weise wird also erreicht, dass der Sperrleiter den elektrischen Widerstand der gesamten Verdampfereinrichtung, nachfolgend auch als Gesamtwiderstand bezeichnet, bis zur Anfangstemperatur nicht oder möglichst geringfügig beeinflusst, und dass der Sperrleiter erst beim Erreichen der Anfangstemperatur einen den Gesamtwiderstand erhöhten Einfluss hat. Mit anderen Worten, der Gesamtwiderstand wird auf diese Weise bis zum Erreichen der Betriebsendtemperatur von der Verdampferkeramik und mit Erreichen der Betriebsendtemperatur von dem zumindest einen Sperrleiter dominiert. In der Folge kann der Betrieb im thermischen Betriebsbereich mit reduziertem Energieaufwand und somit erhöhter Effizienz erfolgen. Zugleich erfolgt beim Erreichen der Betriebsendtemperatur eine genau definierte und zuverlässige Unterbrechung oder zumindest Reduzierung der elektrischen Versorgung. Prinzipiell kann die Betriebsendtemperatur beliebig zwischen der Anfangstemperatur und der Endtemperatur, vorteilhaft zwischen der Anfangstemperatur und der Nenntemperatur, des Kaltleiters liegen.

Vorteilhaft ist es, wenn die Betriebsendtemperatur der Anfangstemperatur des Kaltleiters entspricht. Somit wird insbesondere erreicht, dass zum Erreichen der Betriebsendtemperatur kein erhöhter Energieaufwand, insbesondere kein erhöhter Stromverbrauch, notwendig ist. Dies führt zu einer erhöhten Effizienz der Verdampfereinrichtung. Zudem lässt sich die Verdampfereinrichtung auf diese Weise mit Batterien, insbesondere wiederaufladbaren Batterien, einfach und mit einer erhöhten Betriebsdauer betreiben. Ferner führt dies dazu, dass der Sperrleiter im Betriebsbereich keine oder möglichst keine Wärme erzeugt. Somit erfolgt eine verbesserte Kontrolle über die Verdampfungsparameter.

Der jeweilige zumindest eine Sperrleiter kann prinzipiell beliebig im Strompfad angeordnet sein, sofern er wärmeübertragend mit der Verdampferkeramik verbunden ist.

Denkbar ist es insbesondere, zumindest einen der wenigstens einen Sperrleiter zwischen dem Verdampfer und einem der Anschlüsse anzuordnen. Dies erlaubt eine einfache und kompakte Ausbildung der Verdampfereinrichtung.

Prinzipiell kann die wärmeübertragende Verbindung zwischen dem jeweiligen Sperrleiter und der Verdampferkeramik beliebig ausgestaltet sein.

Besonders bevorzugt sind Ausführungsformen, bei denen zumindest einer der wenigstens einen Sperrleiter, vorteilhaft der jeweilige Sperrleiter, flächig auf der Verdampferkeramik aufliegt. Insbesondere kann einer der wenigstens einen Sperrleiter unmittelbar flächig auf der Verdampferkeramik aufliegen. Dies führt zu einer einfachen und kompakten Ausbildung der Verdampfereinrichtung, wobei zugleich eine einfache und zuverlässige Wärmeübertragung von der Verdampferkeramik auf den Sperrleiter gegeben ist. Zugleich ist es auf diese Weise möglich, den Sperrleiter auf einfache Weise im Strompfad anzuordnen.

Die Verdampferkeramik ist vorteilhaft einstückig und zusammenhängend ausgebildet. Bevorzugt ist es hierbei, wenn an zumindest einer Außenseite der Verdampferkeramik ein Sperrleiter angeordnet ist. Somit ist eine kompakte und einfache Herstellung und Bauweise der Verdampfereinrichtung möglich.

Denkbar ist es auch, die Verdampferkeramik zwei- oder mehrteilig auszubilden. Die Verdampferkeramik kann also zwei voneinander separate Verdampferkörper aufweisen. Dabei kann zwischen wenigstens zwei der zumindest zwei Verdampferkörper ein Sperrleiter angeordnet sein.

Der Verdampfer kann prinzipiell neben der Verdampferkeramik auch weitere Bestandteile aufweisen, welche insbesondere der Führung des elektrischen Stroms dienen können.

Bevorzugt sind Ausführungsformen, bei denen der Verdampfer aus der Verdampferkeramik besteht, also ausschließlich die Verdampferkeramik aufweist. Dies führt zu einer vereinfachten Herstellung der Verdampfereinrichtung und zugleich einer genaueren und/oder einfachen Kontrolle über die Verdampfungsparameter, wie beispielsweise der Definition des Volumens zur Aufnahme der zu verdampfenden Substanz und/oder der erzeugten Wärme.

Der jeweilige zumindest eine Sperrleiter kann prinzipiell aus einem beliebigen Material bzw. Werkstoff hergestellt sein, sofern er beim Überschreiten der Betriebsendtemperatur einen sprunghaft ansteigenden elektrischen Widerstand aufweist.

Vorstellbar ist es insbesondere, dass zumindest einer der wenigstens einen Sperrleiter eine Keramik ist.

Zweckmäßig ist der zumindest eine Sperrleiter derart bemessen, dass er, im Vergleich zur Verdampferkeramik, volumenmäßig einen geringeren Anteil der Verdampfereinrichtung ausmacht. Dies erlaubt insbesondere eine kompaktere Ausbildung der Verdampfereinrichtung. Zudem wird auf diese Weise das zum Bevorraten der Substanz vorgesehene Gesamtvolumen durch die Verdampferkeramik definiert oder zumindest dominiert.

Bevorzugt sind Ausführungsformen, bei denen zumindest einer der wenigstens einen Sperrleiter als eine Lage ausgebildet ist. Insbesondere weist der zumindest eine Sperrleiter auf diese Weise ein im Vergleich zur Verdampferkeramik erheblich reduziertes Volumen auf.

Die jeweilige Lage kann prinzipiell beliebig ausgestaltet sein. Insbesondere kann wenigstens eine der zumindest einen Lage als eine Folie, eine Beschichtung und dergleichen ausgestaltet sein.

Wie vorstehend beschrieben ist es bevorzugt, wenn der elektrische Gesamtwiderstand der Verdampfereinrichtung im thermischen Betriebsbereich vom Verdampfer, insbesondere von der Verdampferkeramik, und oberhalb des Betriebsbereichs, das heißt beim Überschreiten der Betriebsendtemperatur, vom Sperrleiter dominiert ist.

Bevorzugt ist dies derart realisiert, dass der elektrische Widerstand des Sperrleiters im Betriebsbereich maximal der Hälfte des elektrischen Widerstands des Verdampfers, insbesondere der Verdampferkeramik, entspricht.

Der elektrische Widerstand des zumindest einen Sperrleiters setzt sich insbesondere aus dem spezifischen Widerstand sowie dem Volumen bzw. der Strecke entlang des Strompfads zusammen. Dementsprechend kann eine Reduzierung des elektrischen Widerstands des Sperrleiters im Betriebsbereich durch eine Reduzierung des relativen Volumens des Sperrleiters in der Verdampfereinrichtung erreicht werden.

Der Verdampfer, insbesondere die Verdampferkeramik, weist vorzugsweise einen elektrischen Widerstand auf, der bis zur Betriebsendtemperatur, insbesondere im Vergleich zum Anstieg des Widerstands des Sperrleiters ab der Betriebsendtemperatur, geringfügig ansteigt. Bevorzugt weist der elektrische Widerstand des Verdampfers, insbesondere der Verdampferkeramik, einen im Betriebsbereich temperaturabhängigen Verlauf derart aufweist, dass der Widerstand mit der Temperatur maximal um eine Zehnerpotenz steigt.

Bevorzugt sind Ausführungsformen, bei denen der zumindest eine Sperrleiter ein Volumen, nachfolgend auch als Sperrvolumen bezeichnet, aufweist, welches maximal 1/10 (ein Zehntel) des Volumens des Verdampfers, insbesondere der Verdampferkeramik, nachfolgend auch als Verdampfervolumen bezeichnet, beträgt. Das deutlich höhere Verdampfervolumen im Vergleich zum Sperrvolumen führt insbesondere dazu, dass die zu verdampfende Substanz ausschließlich oder zumindest überwiegend in der Verdampferkeramik aufgenommen und gespeichert wird. Mit anderen Worten, der Sperrleiter hat im Vergleich zur Verdampferkeramik keine oder zumindest eine vernachlässigbare Speicherfunktion der Substanz. Somit lässt sich eine verbesserte Kontrolle über die Verdampfungsparameter erreichen. Zugleich ist es auf diese Weise möglich, wie vorstehend erläutert, einen geringeren Einfluss des Widerstands des zumindest einen Sperrleiters auf den Gesamtwiderstand der Verdampfereinrichtung im Betriebsbereich zu erreichen.

Es versteht sich, dass die Verdampfereinrichtung auch drei oder mehr elektrische Anschlüsse aufweisen kann, wobei der Strompfad zwischen zwei der Anschlüsse und die Verdampferkeramik führt.

Die Verdampfereinrichtung kommt bevorzugt in einem Inhalator zum Einsatz, um bei elektrischer Versorgung die Substanz zu verdampfen.

Bevorzugt handelt es sich bei dem Inhalator um einen mobilen und händisch tragbaren Inhalator, der also mitgetragen werden kann. Die vorstehend beschriebenen Vorteile der Verdampfereinrichtung erlauben dabei eine kompakte Ausbildung des Inhalators bei zugleich reduziertem Energieverbrauch und genauerer Kontrolle der Verdampfungsparameter.

Der Inhalator lässt sich, wie vorstehend beschrieben, diskontinuierlich und/oder kontinuierlich betreiben. Entsprechend ist der Inhalator ausgestaltet.

Insbesondere ist es möglich, eine vorgegebene Dosis der Substanz kontrolliert, insbesondere vollständig, zu verdampfen.

Der Inhalator, insbesondere der Verdampfer, kann prinzipiell zum Verdampfen beliebiger Substanzen zum Einsatz kommen. Insbesondere ist es möglich, den Inhalator zum Verdampfen von medizinischen Wirkstoffen enthaltenden Substanzen einzusetzen. Insbesondere erlauben die definierte und/oder kontrollierbare Dosierung eine entsprechend genaue Dosierung der Wirkstoffzuführung zu einem Patienten. Ebenso ist es vorstellbar, den Inhalator bzw. die Verdampfereinrichtung als bzw. in einer elektrischen Zigarette einzusetzen.

Der Inhalator weist vorteilhaft neben der Verdampfereinrichtung eine Elektronik zum Versorgen der Verdampfereinrichtung auf, welche beispielsweise elektrisch mit den Anschlüssen der Verdampfereinrichtung verbunden ist. Dabei kann die Elektronik eine elektrische Verbindung zwischen den Anschlüssen und einer elektrischen Energiequelle, vorzugsweise einer wiederaufladbaren Batterie, des Inhalators, herstellen und trennen. Die Elektronik kann also eine Steuerungseinrichtung zum Steuern des Inhalators umfassen, welche zum Steuern des Inhalators ausgestaltet ist.

Der Inhalator, insbesondere die Elektronik, können dabei, aufgrund der mittels des zumindest einen Sperrleiters gegebenen Unterbrechung oder zumindest Reduzierung der elektrischen Versorgung der Verdampferkeramik beim Überschreiten der Betriebsendtemperatur, frei von entsprechender Regelungselektronik und/oder entsprechender Sensorik sein.

Es versteht sich dabei, dass neben der Verdampfereinrichtung auch ein Inhalator mit einer solchen Verdampfereinrichtung zum Umfang dieser Erfindung gehört.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.

Es zeigen, jeweils schematisch
- Fig. 1: eine isometrische Ansicht einer Verdampfereinrichtung,
- Fig. 2: eine stark vereinfachte, schaltplanartige Darstellung eines Inhalators mit der Verdampfereinrichtung,
- Fig. 3: eine isometrische Ansicht der Verdampfereinrichtung bei einem anderen Ausführungsbeispiel.

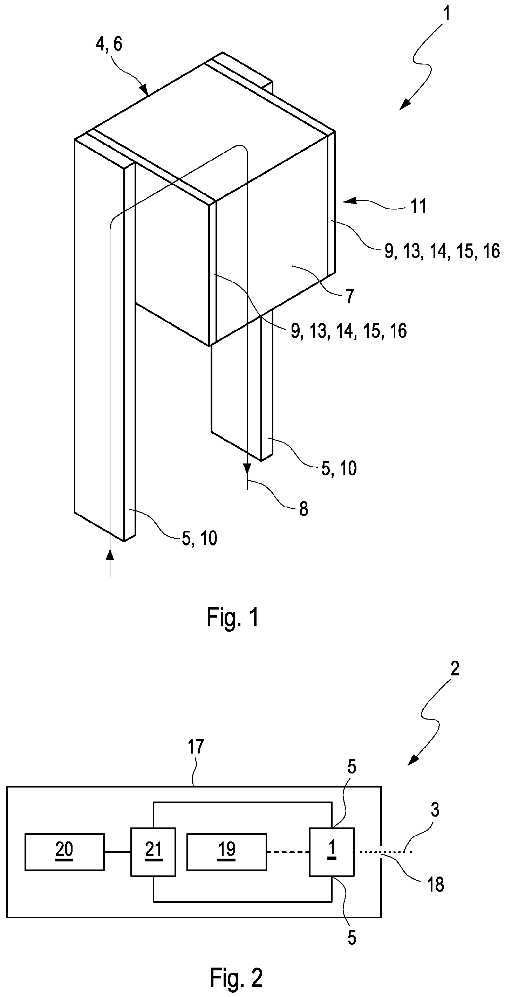

Eine Verdampfereinrichtung 1, wie sie beispielsweise in den Figuren 1 bis 3 gezeigt ist, kommt in einem Inhalator 2, wie er vereinfacht und beispielhaft in Figur 2 gezeigt ist, zum Einsatz. Bei dem Inhalator 2 handelt es sich um einen mobilen und händisch tragbaren Inhalator 2, der im Gebrauch von einem nicht gezeigten Nutzer händisch umgriffen und tragbar ist. Entsprechend ist der Inhalator 2 hinsichtlich seiner Abmessungen ausgebildet.

Die Verdampfereinrichtung 1 dient dem Verdampfen einer Substanz, insbesondere einer vorgegebenen Dosis der Substanz (nicht gezeigt). Bei der Substanz handelt es sich beispielsweise um eine solche, welche einen medizinischen Wirkstoff beinhalten kann, sodass beim Verdampfen ein den Wirkstoff enthaltender Dampf 3 (s. Figur 2) ausgegeben wird, der von einem Nutzer inhaliert wird.

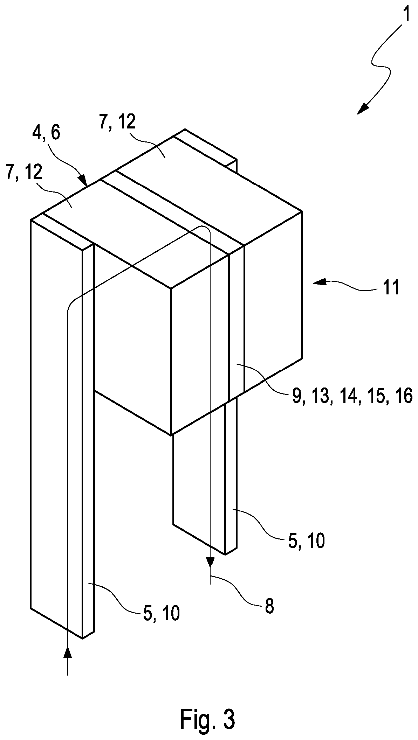

Entsprechend den Figuren 1 und 3 weist die Verdampfereinrichtung 1 einen Verdampfer 4 sowie zwei elektrische Anschlüsse 5 zur elektrischen Versorgung des Verdampfers 4 auf. Der Verdampfer 4 dient sowohl der Aufnahme und dem Speichern der zu verdampfenden Substanz als auch der Erzeugung von Wärme zwecks Verdampfens der Substanz. Zu diesem Zweck weist der Verdampfer 4 eine elektrisch leitfähige Keramik 6 auf, welche nachfolgend auch als Verdampferkeramik 6 bezeichnet wird. In den gezeigten Ausführungsbeispielen besteht der Verdampfer 4 aus der Verdampferkeramik 6. Die Verdampferkeramik 6 weist zum Aufnehmen und Speichern der zu verdampfenden Substanz eine Aufnahmestruktur 7 auf, welche bevorzugt Poren (nicht gezeigt) aufweist, sodass die zu verdampfende Substanz in den Poren aufgenommen wird. Vorteilhaft besteht die Aufnahmestruktur 7 aus dem Poren, ist also eine Porenstruktur. Insbesondere kann die Verdampferkeramik 6 zumindest ein Metalloxid enthalten. Zum Verdampfen der Substanz ist die Verdampferkeramik 6 in einem thermischen Bereich, der nachfolgend auch als Betriebsbereich bezeichnet wird, betrieben. Der Betriebsbereich wird von einer niedrigen Temperatur, nachfolgend auch als Betriebsanfangstemperatur bezeichnet, und von einer hohen Temperatur, nachfolgend auch als Betriebsendtemperatur bezeichnet, begrenzt. Das heißt, dass das Verdampfen der zu verdampfenden und in den Poren aufgenommenen Substanz im Betriebsbereich und somit zwischen der Betriebsanfangstemperatur und der Betriebsendtemperatur erfolgt.

Zum Erzeugen von Wärme wird der Verdampfer 4 mittels der Anschlüsse 5 elektrisch versorgt, sodass ein in den Figuren 1 und 3 angedeuteter Pfad 8 des elektrischen Stroms zwischen den Anschlüssen 5 und durch den Verdampfer 4 führt. Bei elektrischer Versorgung erzeugt die Verdampferkeramik 6 mittels ihres elektrischen Widerstands Wärme zum Verdampfen der Substanz. In diesem Pfad 8, nachfolgend auch als Strompfad 8 bezeichnet, ist zumindest ein Sperrleiter 9 angeordnet, derart, dass der Strompfad 8 zwingend durch den Sperrleiter 9 führt. In den gezeigten Ausführungsbeispielen ist dies dadurch erreicht, dass der zumindest eine Sperrleiter 9 zwischen den Anschlüssen 5 angeordnet ist. Der zumindest eine Sperrleiter 9 ist dabei wärmeübertragend mit der Verdampferkeramik 6 verbunden. In den gezeigten Ausführungsbeispielen ist die wärmeübertragende Verbindung des zumindest einen Sperrleiters 9 mit der Verdampferkeramik 6 durch eine flächige Anordnung des Sperrleiters 9 auf der Verdampferkeramik 6 realisiert. Insbesondere liegt der Sperrleiter 9 unmittelbar an der Verdampferkeramik 6 an. Somit entspricht die Temperatur des zumindest einen Sperrleiters 9 der Temperatur der Verdampferkeramik 6. Der zumindest eine Sperrleiter 9 ist derart ausgestaltet, dass er beim Überschreiten der Betriebsendtemperatur einen sprunghaft ansteigenden elektrischen Widerstand aufweist. Unterhalb der Betriebsendtemperatur ist der zumindest eine Sperrleiter 9 also elektrisch leitend, sodass die Verdampferkeramik 6 bei elektrischer Versorgung im Betriebsbereich betrieben ist, das heißt Temperaturen bis zur Betriebsendtemperatur erreicht. Der sprunghafte Anstieg des elektrischen Widerstands des zumindest einen Sperrleiters 9 führt dazu, dass der durch die Verdampferkeramik 6 strömende elektrische Strom beim Überschreiten der Betriebsendtemperatur unterbrochen oder erheblich reduziert wird, sodass der sprunghafte Anstieg des elektrischen Widerstands des zumindest einen Sperrleiters 9 die Betriebsendtemperatur definiert oder zumindest dominiert. Somit ist es möglich, die Verdampfereinrichtung 1 mit kontrollierten Verdampfungsparametern zu betreiben. Dies erlaubt es insbesondere, eine vorgegebene Menge der zu verdampfenden Substanz und somit eine vorgegebene Dosis der Substanz zu verdampfen. Dabei ist hierzu eine separate Steuerungselektronik (nicht gezeigt) und/oder separate Sensorik (nicht gezeigt), beispielsweise zum Ermitteln der Temperatur der Verdampferkeramik, nicht notwendig.

In den gezeigten Ausführungsbeispielen sind die elektrischen Anschlüsse 5 jeweils als eine Leiterplatte 10, beispielsweise aus einem Metall oder einer Metalllegierung, ausgebildet. Der Verdampfer 4 ist zwischen den Anschlüssen 5 angeordnet.

In den gezeigten Ausführungsbeispielen bilden der Verdampfer 4, insbesondere die Verdampferkeramik 6, und der zumindest eine Sperrleiter 9 ein zusammenhängendes Modul 11, welches zwischen den Anschlüssen 5 angeordnet ist. In den gezeigten Ausführungsbeispielen weist das Modul 11 eine quaderförmige Ausbildung auf. Wie den Figuren 1 und 3 entnommen werden kann, ist hierbei ein Volumenanteil der Verdampferkeramik 6 im Gesamtvolumen des Moduls 11 erheblich größer als ein Volumenanteil des zumindest einen Sperrleiters 9. Insbesondere ist der Volumenanteil des zumindest einen Sperrleiters 9, nachfolgend auch als Sperrvolumen bezeichnet, maximal 1/10 des Volumenanteils der Verdampferkeramik 6, nachfolgend auch als Verdampfervolumen bezeichnet. Dies führt insbesondere dazu, dass das Volumen zum Aufnehmen der Substanz von der Verdampferkeramik 6 bestimmt oder zumindest dominiert ist. Zudem führt dies dazu, dass der zumindest eine Sperrleiter 9 im Betriebsbereich eine vernachlässigbare Rolle beim elektrischen Gesamtwiderstand des Moduls 11 des Verdampfers 4 spielt. Mit anderen Worten, der elektrische Gesamtwiderstand des Verdampfers 4 wird im Betriebsbereich von der Verdampferkeramik 6 dominiert, wohingegen er oberhalb des Betriebsbereichs von dem zumindest einen Sperrleiter 9 dominiert wird.

Beim in Figur 1 gezeigten Ausführungsbeispiel ist die Verdampferkeramik 6 zusammenhängend und quaderförmig ausgebildet, wobei zwischen der jeweiligen, einem der Anschlüsse 4 zugewandten Außenseite der Verdampferkeramik 6 und dem zugehörigen Anschluss 5 ein Sperrleiter 9 angeordnet ist.

Das in Figur 3 gezeigte Ausführungsbeispiel unterscheidet sich von dem in Figur 1 gezeigten Ausführungsbeispiel dadurch, dass die Verdampferkeramik 6 zweiteilig ausgebildet ist und somit zwei Verdampferkörper 12 aufweist, welche im gezeigten Ausführungsbeispiel jeweils identisch und quaderförmig ausgebildet sind. Hierbei ist in dem gezeigten Ausführungsbeispiel ein einziger Sperrleiter 9 vorgesehen, der zwischen den Verdampferkörpern 12 angeordnet ist.

In den gezeigten Ausführungsbeispielen ist der jeweilige Sperrleiter 9 als eine im Vergleich zur Verdampferkeramik 6 bzw. zu den Verdampferkörpern 12 dünne Lage 13 ausgebildet und kann daher auch als Sperrlage 14 bezeichnet werden.

Der jeweilige Sperrleiter 9 ist vorzugsweise ein Kaltleiter 15, welcher ab einer Anfangstemperatur einen sprunghaften und um mehrere Zehnerpotenzen steigenden elektrischen Widerstand aufweist. Hierbei entspricht die Betriebsendtemperatur vorteilhaft einer Temperatur zwischen der Anfangstemperatur und einer Endtemperatur des Kaltleiters 15, insbesondere der Anfangstemperatur des Kaltleiters 15.

Insbesondere ist der Kaltleiter 15 eine sich von der Verdampferkeramik 6 unterscheidende Keramik 16, welche nachfolgend auch als Sperrkeramik 16 bezeichnet wird. Durch das geringere Sperrvolumen der Sperrkeramik 16 im Vergleich zum Verdampfervolumen der Verdampferkeramik 6 wird dabei die Gesamtaufnahmefähigkeit des Verdampfers 4 von der Verdampferkeramik 6 bestimmt oder zumindest dominiert.

Wie Figur 2 entnommen werden kann, handelt es sich bei dem Inhalator 2 um einen händisch tragbaren Inhalator 2, bei dem die Verdampfereinrichtung 1 in einem Gehäuse 17 aufgenommen ist, welches eine Auslassöffnung 18 zum Auslassen des Mittels des Verdampfers 4 erzeugten Dampfs 3 aufweist. Der Inhalator 2 des gezeigten Ausführungsbeispiels weist ferner einen, vorzugsweise nachfüllbaren oder austauschbaren, Behälter 19 zum Bevorraten der zu verdampfenden Substanz auf, der, wie mit einer gestrichelten Linie angedeutet, fluidisch mit dem Verdampfer 4, insbesondere der Verdampferkeramik 6, verbunden oder verbindbar ist.

Die Verdampfereinrichtung 1 und der Behälter 19 können hierbei eine Einheit bilden, welche austauschbar im Inhalator 2 aufgenommen ist.

Alternativ ist es möglich, dass der Behälter 19 im Inhalator 2 dauerhaft aufgenommen und nachfüllbar ist. In diesem Fall kann auch die Verdampfereinrichtung 1 dauerhaft im Inhalator 2 aufgenommen sein.

Alternativ ist es möglich, dass der Behälter 19 austauschbar ist. In diesem Fall kann auch die Verdampfereinrichtung 1 dauerhaft im Inhalator 2 aufgenommen sein.

Der Inhalator 2 weist ferner eine wiederaufladbare Batterie 20 zur elektrischen Versorgung der Verdampfereinrichtung 1 sowie eine mit der Verdampfereinrichtung 1 elektrisch verbundene Elektronik 21 auf. Die Elektronik 21 ist derart mit der Batterie 20 verbunden, dass sie die elektrische Verbindung der Batterie 20 mit der Verdampfereinrichtung 1 zwecks elektrischer Versorgung der Verdampfereinrichtung 1 herstellen und unterbrechen kann.

## Patentansprüche

1. Verdampfereinrichtung (1) zum Verdampfen einer Substanz, insbesondere für einen Inhalator (2),
- mit einem Verdampfer (4) zum Aufnehmen und Verdampfen der Substanz,
- wobei der Verdampfer (4) eine elektrisch leitfähige Verdampferkeramik (6) mit einer Aufnahmestruktur (7) aufweist, die integral in der Verdampferkeramik ausgebildet und/oder ausgeformt ist, sodass im Betrieb die zu verdampfende Substanz in der Aufnahmestruktur (7) aufgenommen ist,
- mit zwei elektrischen Anschlüssen (5) zur elektrischen Versorgung des Verdampfers (4), wobei ein elektrischer Strompfad (8) zur elektrischen Versorgung des Verdampfers (4) durch die Anschlüsse (5) und durch die Verdampferkeramik (6) verläuft, wobei,
- die Verdampferkeramik (6) derart ausgestaltet ist, dass sie im Betrieb bei elektrischer Versorgung zum Verdampfen der in der Aufnahmestruktur (7) aufgenommenen Substanz homogen Wärme in einem thermischen Betriebsbereich zwischen einer Betriebsanfangstemperatur und einer Betriebsendtemperatur erzeugt,
- die Verdampfereinrichtung (1) zumindest einen im Strompfad (8) angeordneten Sperrleiter (9) aufweist, welcher wärmeübertragend mit der Verdampferkeramik (6) verbunden ist,
- der zumindest eine Sperrleiter (9) derart ausgestaltet ist, dass er beim Überschreiten der Betriebsendtemperatur einen sprunghaft ansteigenden elektrischen Widerstand aufweist, und
- zumindest einer der wenigstens einen Sperrleiter (9) flächig auf der Verdampferkeramik (6) aufliegt.

2. Verdampfereinrichtung nach Anspruch 1,
**dadurch gekennzeichnet,**
- **dass** zumindest einer der wenigstens einen Sperrleiter (9) als ein Kaltleiter (15) ausgestaltet ist,
- **dass** die Betriebsendtemperatur zwischen einer Anfangstemperatur und einer Endtemperatur des zumindest einen Kaltleiters (15) liegt.

3. Verdampfereinrichtung nach Anspruch 2,
**dadurch gekennzeichnet,**
**dass** die Betriebsendtemperatur der Anfangstemperatur entspricht.

4. Verdampfereinrichtung nach einem der Ansprüche 1 bis 3,
**dadurch gekennzeichnet,**
**dass** zumindest einer der wenigstens einen Sperrleiter (9) zwischen dem Verdampfer (4) und einem der Anschlüsse (5) angeordnet ist.

5. Verdampfereinrichtung nach einem der Ansprüche 1 bis 4,
**dadurch gekennzeichnet,**
- **dass** die Verdampferkeramik (6) zwei benachbarte Verdampferkörper (12) aufweist,
- **dass** zwischen den Verdampferkörpern (12) eine der zumindest einen Sperrleiter (9) angeordnet ist, sodass die Verdampferkörper (12) und der Sperrleiter (9) einteilig ausgebildet sind.

6. Verdampfereinrichtung nach einem der Ansprüche 1 bis 5,
**dadurch gekennzeichnet,**
**dass** zumindest einer der wenigstens einen Sperrleiter (9) als eine Lage (13) ausgebildet ist.

7. Verdampfereinrichtung nach einem der Ansprüche 1 bis 6,
**dadurch gekennzeichnet,**
**dass** die Aufnahmestruktur (7) Poren zur Aufnahme der Substanz aufweist.

8. Verdampfereinrichtung nach einem der Ansprüche 1 bis 7,
**dadurch gekennzeichnet,**
**dass** der zumindest eine Sperrleiter (9) derart ausgestaltet ist, dass der elektrische Widerstand des Sperrleiters (9) im Betriebsbereich maximal der Hälfte des elektrischen Widerstands des Verdampfers (4) entspricht.

9. Verdampfereinrichtung nach einem der Ansprüche 1 bis 8,
**dadurch gekennzeichnet,**
**dass** der elektrische Widerstand des Verdampfers (4) im Betriebsbereich maximal um eine Zehnerpotenz steigt.

10. Verdampfereinrichtung nach einem der Ansprüche 1 bis 9,
**dadurch gekennzeichnet,**
**dass** ein Sperrvolumen des zumindest einen Sperrleiters (9) maximal ein Zehntel eines Verdampfervolumens der Verdampferkeramik (6) beträgt.

11. Verdampfereinrichtung nach einem der Ansprüche 1 bis 10,
**dadurch gekennzeichnet,**
**dass** der Verdampfer (4) aus der Verdampferkeramik (6) besteht.

12. Inhalator (2), insbesondere händisch tragbarer Inhalator (2), zum Verdampfen einer Substanz, mit einer Verdampfereinrichtung (1) nach einem der Ansprüche 1 bis 11 und mit einer Elektronik (21), welche elektrisch mit den Anschlüssen (5) verbunden ist.

## Claims

1. Evaporator device (1) for evaporating a substance, in particular for an inhaler (2),
- comprising an evaporator (4) for receiving and evaporating the substance,
- wherein the evaporator (4) has an electrically conductive evaporator ceramic (6) with a receiving structure (7) that is integrally formed and/or shaped in the evaporator ceramic, such that during operation the substance to be evaporated is received in the receiving structure (7),
- comprising two electrical connections (5) for electrical supply of the evaporator (4), wherein an electrical current path (8) for electrical supply of the evaporator (4) extends through the connections (5) and through the evaporator ceramic (6), wherein
- the evaporator ceramic (6) is configured such that, during operation and upon electrical supply for evaporating the substance received in the receiving structure (7), it homogeneously generates heat in a thermal operating range between an operation starting temperature and an operation end temperature,
- the evaporator device (1) has at least one blocking conductor (9) arranged in the current path (8) and heat-transmittingly connected to the evaporator ceramic (6),
- the at least one blocking conductor (9) is configured such that, upon exceeding the operation end temperature, it has an abruptly increasing electrical resistance, and
- at least one of the at least one blocking conductors (9) lies flat on the evaporator ceramic (6).

2. Evaporator device according to claim 1,
**characterized in that**
- at least one of the at least one blocking conductors (9) is configured as a PTC thermistor (15), and
- the operation end temperature lies between a starting temperature and an end temperature of the at least one PTC thermistor (15).

3. Evaporator device according to claim 2,
**characterized in that**
the operation end temperature corresponds to the starting temperature.

4. Evaporator device according to any one of claims 1 to 3,
**characterized in that**
at least one of the at least one blocking conductors (9) is arranged between the evaporator (4) and one of the connections (5).

5. Evaporator device according to any one of claims 1 to 4,
**characterized in that**
- the evaporator ceramic (6) has two adjacent evaporator bodies (12),
- and **in that** one of the at least one blocking conductors (9) is arranged between the evaporator bodies (12) such that the evaporator bodies (12) and the blocking conductor (9) are formed integrally.

6. Evaporator device according to any one of claims 1 to 5,
**characterized in that**
at least one of the at least one blocking conductors (9) is formed as a layer (13).

7. Evaporator device according to any one of claims 1 to 6,
**characterized in that**
the receiving structure (7) has pores for receiving the substance.

8. Evaporator device according to any one of claims 1 to 7,
**characterized in that**
the at least one blocking conductor (9) is configured such that the electrical resistance of the blocking conductor (9) in the operating range corresponds to at most half of the electrical resistance of the evaporator (4).

9. Evaporator device according to any one of claims 1 to 8,
**characterized in that**
the electrical resistance of the evaporator (4), in the thermal operating range, increases at most by a power of ten.

10. Evaporator device according to any one of claims 1 to 9,
**characterized in that**
a blocking volume of the at least one blocking conductor (9) amounts at most to a tenth of an evaporator volume of the evaporator ceramic (6).

11. Evaporator device according to any one of claims 1 to 10,
**characterized in that**
the evaporator (4) consists of the evaporator ceramic (6).

12. Inhaler (2), in particular a manually portable inhaler (2), for evaporating a substance, comprising an evaporator device (1) according to any one of claims 1 to 11, and comprising electronics (21) electrically connected to the connections (5).

## Revendications

1. Dispositif évaporateur (1) pour l'évaporation d'une substance, en particulier pour un inhalateur (2),
- avec un évaporateur (4) pour la réception et l'évaporation de la substance,
- dans lequel l'évaporateur (4) présente une céramique d'évaporateur (6) électroconductrice avec une structure de réception (7) qui est formée et/ou moulée d'un seul tenant dans la céramique d'évaporateur de sorte qu'en fonctionnement, la substance à évaporer soit reçue dans la structure de réception (7),
- avec deux raccords (5) électriques pour l'alimentation électrique de l'évaporateur (4), dans lequel un trajet de courant (8) électrique s'étend pour l'alimentation électrique de l'évaporateur (4) à travers les raccords (5) et la céramique d'évaporateur (6), dans lequel
- la céramique d'évaporateur (6) est configurée de manière à générer en fonctionnement lors de l'alimentation électrique pour l'évaporation de la substance reçue dans la structure de réception (7) de manière homogène de la chaleur dans une plage de fonctionnement thermique entre une température de début de fonctionnement et une température de fin de fonctionnement,
- le dispositif évaporateur (1) présente au moins un conducteur de blocage (9) agencé dans le trajet de courant (8), conducteur qui est relié de manière à transmettre la chaleur à la céramique d'évaporateur (6),
- le au moins un conducteur de blocage (9) est configuré de manière à présenter lors du dépassement de la température de fin de fonctionnement une résistance électrique qui augmente brusquement et
- au moins un des au moins un conducteur de blocage (9) repose sur la surface sur la céramique d'évaporateur (6).

2. Dispositif évaporateur selon la revendication 1,
**caractérisé en ce que**
- au moins un des au moins un conducteur de blocage (9) est configuré comme une résistance CTP (15),
- la température de fin de fonctionnement se situe entre une température de début et une température de fin de l'au moins une résistance CTP (15).

3. Dispositif évaporateur selon la revendication 2,
**caractérisé en ce que**
la température de fin de fonctionnement correspond à la température de début.

4. Dispositif évaporateur selon l'une quelconque des revendications 1 à 3,
**caractérisé en ce que**
au moins un des au moins un conducteur de blocage (9) est agencé entre l'évaporateur (4) et l'un des raccords (5).

5. Dispositif évaporateur selon l'une quelconque des revendications 1 à 4,
**caractérisé en ce que**
- la céramique d'évaporateur (6) présente deux corps d'évaporateur (12) contigus,
- un des au moins un conducteur de blocage (9) est agencé entre les corps d'évaporateur (12) de sorte que les corps d'évaporateur (12) et le conducteur de blocage (9) soient formés d'un seul tenant.

6. Dispositif évaporateur selon l'une quelconque des revendications 1 à 5,
**caractérisé en ce que**
au moins un des au moins un conducteur de blocage (9) est formé comme une couche (13).

7. Dispositif évaporateur selon l'une quelconque des revendications 1 à 6,
**caractérisé en ce que**
la structure de réception (7) présente des pores pour la réception de la substance.

8. Dispositif évaporateur selon l'une quelconque des revendications 1 à 7,
**caractérisé en ce que**
le au moins un conducteur de blocage (9) est configuré de telle manière que la résistance électrique du conducteur de blocage (9) corresponde dans la plage de fonctionnement au maximum à la moitié de la résistance électrique de l'évaporateur (4).

9. Dispositif évaporateur selon l'une quelconque des revendications 1 à 8,
**caractérisé en ce que**
la résistance électrique de l'évaporateur (4) augmente dans la plage de fonctionnement au maximum d'une puissance de dix.

10. Dispositif évaporateur selon l'une quelconque des revendications 1 à 9,
**caractérisé en ce que**
un volume de blocage de l'au moins un conducteur de blocage (9) s'élève au maximum à un dixième d'un volume d'évaporation de la céramique d'évaporateur (6).

11. Dispositif évaporateur selon l'une quelconque des revendications 1 à 10,
**caractérisé en ce que**
l'évaporateur (4) se compose de la céramique d'évaporateur (6).

12. Inhalateur (2), en particulier inhalateur (2) portable à la main, pour l'évaporation d'une substance, avec un dispositif évaporateur (1) selon l'une quelconque des revendications 1 à 11 et avec une électronique (21) qui est électriquement reliée aux raccords (5).
